# EUROPÄISCHE PATENTSCHRIFT

(11) **EP 1 321 160 B1**
(45) Veröffentlichungstag und Bekanntmachung des Hinweises auf die Patenterteilung: **15.11.2006**
(21) Anmeldenummer: 02028449.3
(22) Anmeldetag: 19.12.2002
(51) Int. Cl.: A61M 15/00, A61M 3/00

(54) **Spender zum Mischen und Austragen von Medien**
Dispenser for mixing and delivering of substances
Distributeur pour mélange et distribution de substances

(30) Priorität: 21.12.2001 DE 10164452
(43) Veröffentlichungstag der Anmeldung: 25.06.2003
(73) Patentinhaber: Ing. Erich Pfeiffer GmbH, 78315 Radolfzell (DE)
(72) Erfinder: Stihl, Alexander, 78315 Radolfzell (DE)
(74) Vertreter: Patentanwälte Ruff, Wilhelm, Beier, Dauster & Partner

(56) Entgegenhaltungen:
- EP-A- 1 186 312
- WO-A-99/46055
- DE-A- 19 708 406
- US-A- 2 151 418
- US-A- 2 754 590
- US-A- 5 349 947

## Beschreibung

Die Erfindung betrifft einen Spender zum Mischen und Austragen eines aus zwei Medien gemischten Mediumsgemischs. Ein Spender gemäß dem Oberbegriff des Anspruchs 1 ist aus WO-A-99/46055 bekannt.

Aus der DE 19817417 A (entsprechend US 09/554031) ist ein Spender bekannt, bei dem ein auszutragendes Medium, beispielsweise ein Pulver, in einem Blisterring aufgenommen ist. Der Benutzer betätigt einen Luftpumpzylinder und drückt dabei den Blisterring gegen einen hohlen Stoßdorn, der eine Abdeckfolie einer Blisterkammer durchstößt. Durch diese Öffnung dringt Luft in die Blisterkammer und trägt das Medium durch den hohlen Stoßdorn aus. Die Luft dringt also neben oder durch den Stoßdorn in die Medienkammer ein und durch diesen wieder hinaus. Dies beschränkt den Leitungsquerschnitt und die Strömungsführung für eine gute Vermischung.

Aus der US 2 151 418 ist ein Pulverzerstäuber bekannt geworden, bei dem eine patronenartige Pulverkartusche in eine zusammenschraubbare Kammer eingesetzt wird. Die Kartusche ist auf beiden Seiten durch Zusammenfalten eines Deckblattes verschlossen, wobei jedoch die eine Seite beim Einsetzen in den Zerstäuberkopf geöffnet wird, so dass sie in Verbindung mit einem Überströmkanal steht, über den Luft von einem flexiblen Zerstäuberball in die Kartusche eingeleitet werden kann. Dabei öffnet sich auch das zusammengefaltete andere Ende der Kartusche und gibt den Austritt frei.

### Aufgabe der Erfindung

Eine Aufgabe der Erfindung ist es, einen Spender zu schaffen, bei dem Führung der Medien vereinfacht, die Anforderung an die leichte Mischbarkeit der Medien verringert und der Mischvorgang verbessert ist.

### Lösung

Wie im Anspruch 1 definiert, schafft die Erfindung einen Spender, bei dem ein erstes Medium, z.B. ein Förderfluid, aus einer ersten Kammer bei Betätigung des Spenders in eine anfänglich verschlossene zweite Kammer dadurch gefördert wird, dass diese durch einen Stoßdorn geöffnet wird. Das unter Druck stehende erste Medium dringt also in die zweite Kammer ein und setzt diese unter Mediendruck, der die Öffnung der zweiten Kammer bewirkt. Das kann durch Sprengen einer Wandung der zweiten Kammer geschehen, beispielsweise der Abdeckfolie eines Blisters, dessen Körper von dem Stoßdorn von unten angestochen wird. Dabei kann dieser Wandungsteil durch Materialschwächung, z.B. nach Art einer Sollbruchstelle, entsprechend vorbereitet sein. Es ist aber auch möglich, dass das Austragverschlusselement der zweiten Kammer ebenfalls angestochen wird, um das Öffnen unter Mediendruck zu unterstützen. Die zweite Kammer kann auch einen verschiebbaren Wandungsteil, beispielsweise einen Verschlusstopfen aufweisen, der durchstochen, abgesprengt oder durch einen Bypass umgangen wird, wenn Mediendruck aus der zweiten Kammer auf ihn einwirkt.

In jedem Falle ist der Medienweg vereinfacht, weil die Durchströmung der zweiten Kammer ohne wesentliche Umlenkung geschehen kann, also ein gerader Durchgang geschaffen wird. Dennoch ist die Mischung der beiden Medien gut, weil das Förderfluid gezielt eindringt und durch den Druckaufbau in der zweiten Kammer auch eine gewisse Mischungszeit zur Verfügung steht, die auch dazu beitragen kann, dass sich das zweite Medium in dem ersten Medium löst, beispielsweise ein Pulver in einer Flüssigkeit.

Das zweite Medium kann in flüssiger oder fester Form, insbesondere als Pulver vorliegen. Es kann dann mit dem ersten Medium, das vorwiegend gasförmig (Luft) oder flüssig sein kann, gemischt werden und so alle möglichen Gemischformen oder Dispersionen zwischen diesen bilden kann, insbesondere Aerosole mit flüssigen oder festen Partikeln, Emulsionen, Lösungen oder Suspensionen sowie Schäume. In der Pharmazie ist dies besonders wichtig, z.B. für Lyophilisate. Viele pharmazeutische Wirkstoffe, die meist das zweite Medium bilden, sind in der Applizierungsform pulverförmig oder in flüssiger Form nicht beständig, so dass sie erst kurz vor dem Applizieren in Mischung oder Lösung gebracht werden können. Die Pharmazeutika sind oft gefriergetrocknete Pulver. Pharmazeutika sind oft über die Nasenschleimhäute absorbierbar oder oberflächenwirksam. Auch das Förderfluid (erstes Medium) kann pharmazeutische Wirkstoffe enthalten bzw. mit dem zweiten Medium zur Bildung des endgültigen Pharmazeutikums chemisch oder physikalisch reagieren.

Wenn als Austragverschlusselement eine Folie verwendet wird, so kann die Materialstärke im Bereich der Schwächung auf ca. 9 - 12 µm Foliendicke minimiert sein und damit unterhalb der Wandstärken der restlichen Folie liegen. Hierbei finden insbesondere Metallfolien, z.B. Aluminiumfolien, Verwendung. Da solche geschwächten Stellen keine Definition des Beginnes einer Bruchstelle enthalten, sollte bei der Auslegung der Folie darauf geachtet werden, dass ein möglichst kontinuierlich fortschreitendes, an einer bestimmten Stelle beginnendes Aufreißen der Folie sichergestellt ist, um z.B. eine Knallbildung zu vermeiden. Hierzu könnte beispielsweise eine Laserperforierung des Materials dienen, die aber auch längs der gesamten Sollbruchstelle geführt sein kann. Die Perforierung wird so vorgenommen, dass das Material auch in ihrem Bereich gegen Fluide und Gasaustausch dicht bleibt.

Ergänzend oder alternativ zur Materialschwächung kann im Bereich des aufbrechenden Oberflächenabschnittes der Stoßdorn den Medienspeicher an- oder durchstechen. Dies definiert genau die Position des Aufreißbeginns.

Vorteilhaft kann die zweite Kammer (Medienspeicher) zumindest bereichsweise verformbar sein. Dadurch wird auch bei wechselnden Außendrücken kaum ein Druck unterschieden zwischen der Atmosphäre und dem Inneren insbesondere der zweiten Kammer erzeugt, was der Fusion und anderen Gasaustausch mit der Atmosphäre gering hält. Dadurch können die Materialdicken der Wandung des Medienspeichers gering sein.

Bei der vorteilhaften Verwendung eines Blisters besteht dieser meist aus einem Formkörper, der z.B. napfförmig mit einem Rand ausgebildet ist und durch Folienmaterial verschlossen wird. Dies kann eine Metallfolie, eine metallbedampfte Kunststofffolie oder eine mehrschichtige, aus Laminaten zusammengesetzte Folie sein. Die Wahl des Folienmaterials bestimmt die "Verdämmung", dass heißt die Größe des Druckaufbaus vor dem druckbedingten Öffnen des Austragverschlusselementes. Diese sollte so groß gewählt werden, dass das austretende Mediengemisch die gewünschte Gebrauchsform hat oder erreichen kann. Bei einer Ausgabe in Form eines Sprühnebels durch eine Sprühdüse sollte ein relativ großer Anfangsdruck vorhanden sein.

Gemäß einer weiteren Ausführungsform weist der Medienspeicher einen um einen Bewegungsweg axial verschiebbaren angeordneten Verschlussstopfen auf. Durch axiales Verschieben wird der Fluidpfad zwischen Medienspeicher und Austragöffnung freigegeben. Dies geschieht unter dem Druckanstieg in der zweiten Kammer.

Die zweite Kammer kann austauschbar sein. Somit kann ein Spender zur wiederholten Benutzung neu geladen werden. Ferner ist es möglich, durch die manuelle Betätigung das Förderfluid in der ersten Kammer anfänglich nur unter Druck zu setzen, ohne die Überströmung in die erste Kammer einzuleiten. Es tritt dann mit großer Geschwindigkeit und entsprechenden Vorteilen für den Mischungsvorgang in die zweite Kammer ein. Auch muss das Förderfluid erst kurz vor dem Austrag komprimiert werden und nicht in einem druckdichten Speicher längere Zeit bevorratet werden, was jedoch auch möglich ist.

Die vorstehenden und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischenüberschriften beschränkt die unter diesen gemachten Aussagen nicht in ihrer Allgemeingültigkeit

### Kurzbeschreibung der Zeichnungen

Ausführungsbeispiele der Erfindung sind in den Zeichnungen dargestellt und werden im folgenden näher erläutert. Es zeigen:
- Fig. 1: ein schematischen Längsschnitt durch einen Spender,
- Fig. 2 bis 3: Medienspeicher (zweite Kammern) in zwei aufeinanderfolgenden Verfahrensstufen, sowie
- Figur 4: ein Längsschnitt durch einen Spender nach der Erfindung.

### Beschreibung der bevorzugten Ausführungsbeispiele

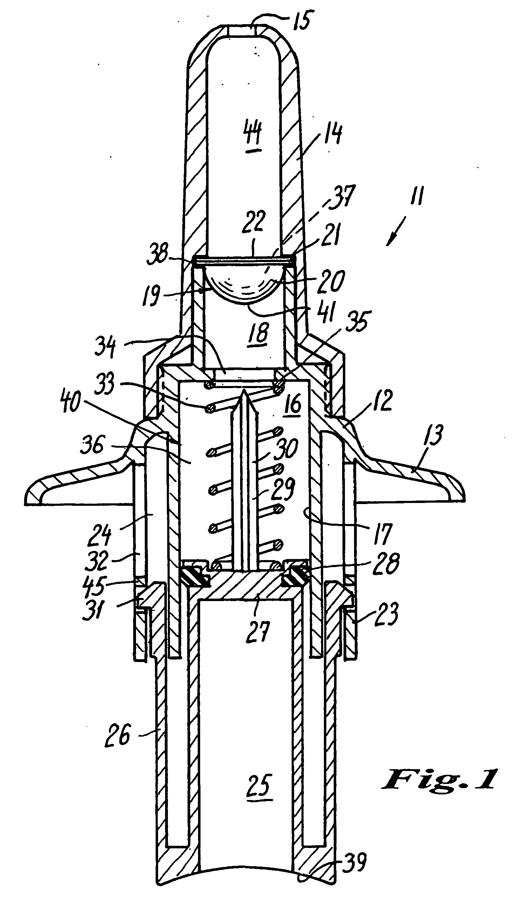

Figur 1 zeigt einen Spender 11 mit einem Basiskörper 12, der seitlich vorstehende Schultern 13 hat, die zur Auflage zweier Finger eines Benutzers geeignet sind. Auf dem Basiskörper 12 ist ein auch als Nasenolive bezeichneter langgestreckter Adapter 14 aufsteckbar oder aufschraubbar, der an seinem abgerundeten Ende eine Austragöffnung 15 aufweist. Diese ist als relativ großes offenes Loch gezeigt, kann aber bei Ausbildung des Spenders als Zerstäuber auch eine Zerstäubungsdüse enthalten.

Der Basiskörper 12 enthält eine erste Kammer 16, die einen als Pumpenzylinder ausgebildeten zylindrischen Abschnitt 17 und einen damit in Verbindung stehenden Abschnitt 18 aufweist. An dessen Ende ist eine zweite Kammer 19 auswechselbar eingesetzt und wird dort vom Adapter gehalten. Die zweite Kammer besteht aus einem Blister mit einem halbkugelig napfförmigen Formteil 20 aus Kunststoff und einem Rand 21, auf dem ein die zweite Kammer dicht versiegelndes Austragverschlusselement 22 in Form einer Metall- oder Kunststofffolie aufgesiegelt ist. Der Blister ist am Rand 21 dicht zwischen einer oberen Stirnfläche des Basisteils und dem Adapter eingespannt.

Der Zylinder 17 des Basisteils 12 wird von einem leicht aufweitbaren Mantel 23 umgeben. In dem dazwischen gebildeten Ringspalt 24 ist ein Betätigungselement 25 geführt, und zwar mit zylindrischen oder stegförmigen Führungsteilen 26. Zwischen diesen enthält das Betätigungselement einen Kolben 27, der mittels einer eingesetzten oder auch angeformten Dichtung 28 im Zylinder 17 läuft. Am Kolbenboden ist ein Stoßdorn 29 angeordnet oder angeformt, der durch eine verrippte Struktur, beispielsweise einem kreuzförmigen Querschnitt in später noch erläuterter Weise Überströmkanäle 30 bildet.

Die Führungsteile oder -arme 26 des Betätigungselements 25 haben an ihren Enden Rastnasen 31, die in Führungsschlitzen 32 des Mantels 23 laufen und verhindern, dass das Betätigungselement 25 sich nach anfänglichen Einschnappen von dem Basisteil 12 löst.

Eine Rückstellfeder 33 ist im Zylinder 17, den Stoßdorn 29 umgebend angeordnet und stützt sich auf die Verbindungsöffnung 34 zwischen Zylinder 17 und anschließendem Abschnitt 18 umgebenden Schultern 35 ab. Es kann eine Stahl-Schraubenfeder oder eine ggf. an dem Kolben 27 angespitzte Kunststoff-Feder sein. In diesem Fall wären alle Teile des Spenders aus Kunststoff, was ein artenreines Recycling ermöglicht.

Die erste Kammer 16 enthält ein erstes Medium 36, das ein Förderfluid, z.B. eine Flüssigkeit oder ein Gas, z.B. Luft ist. Die zweite Kammer (Blister 19) bildet einen Medienspeicher für ein zweites Medium 37, beispielsweise ein pulverförmiges Pharmazeutikum.

### Funktion

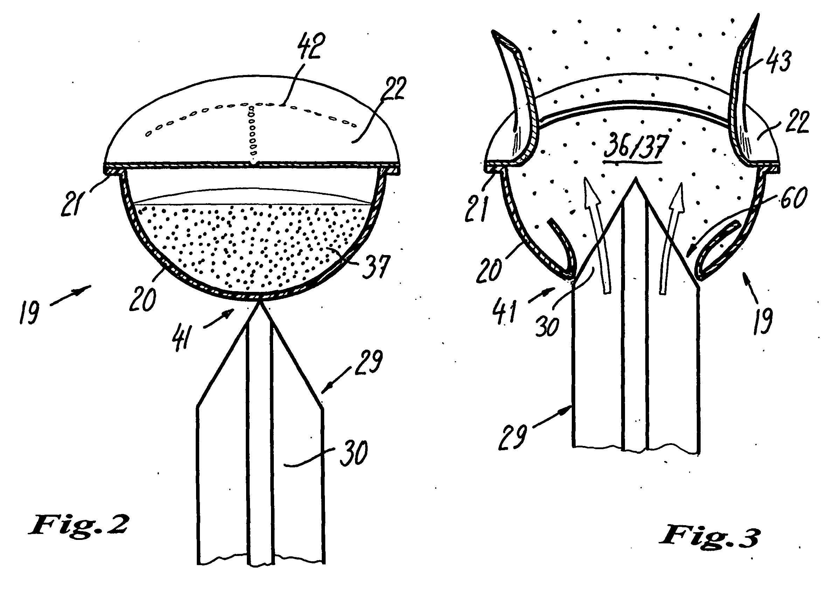

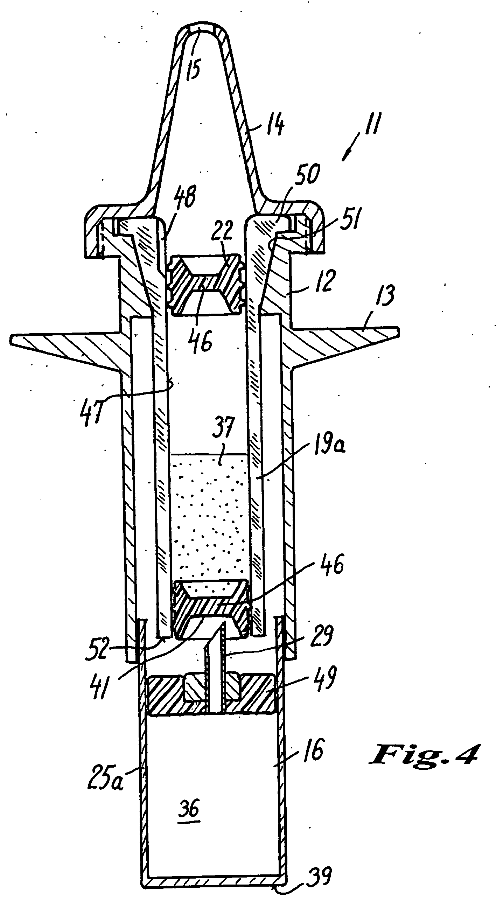

Ein Benutzer legt zur Vorbereitung der Applikation ein Blister 19 in den Spender, indem er den Adapter 14 abnimmt oder abschraubt, den Blister auf die Stirnfläche 38 des Basisteils 12 legt und den Adapter wieder anbringt. Der Zylinder 17 ist dann mit Luft, die in diesem Falle das Förderfluid 36 bildet, gefüllt. Legt der Benutzer nun zwei Finger auf die Schultern 13 und drückt mit dem Daumen auf die Betätigungsfläche 39 am Betätigungselement 25, dann kann er dieses nach oben drücken und damit in der durch den Zylinder 17 und den Kolben 27 gebildeten Pumpe 40 die Luft komprimieren. Dies geschieht so lange, bis der Stoßdorn 29 auf das Formteil 20 des Blisters 19 trifft und dieses durchsticht (siehe Figur 3).

Aus Figur 3 ist zu sehen, dass der in perspektivischer Schnittdarstellung gezeigte Blister am Boden seines Formteils, der ein Überström-Verschlusselement 41 bildet, durchstoßen wird. In Folge der Kreuzstruktur des Stoßdorns 29 bildet sich im Bereich der Überströmkanäle 30 eine Öffnung 60, durch die nun die komprimierte Luft in den Blister eindringt und diesen unter Druck setzt.

Figur 2 zeigt, dass der Blister an seiner das Austragverschlusselement 22 bildenden aufgesiegelten Folie Materialschwächungen 42 aufweist, beispielsweise eine Rillung, Prägung oder nicht durchgängige Laserperforation, die im darstellten Beispiel die Form eines rundlichen H hat. Unter dem in dem Blister entstandenen Mediendruck wird daher die Folie entlang dieser Materialschwächung aufreisen und z.B. in zwei seitlichen Lappen 43 auseinander klappen bzw. bei einer Materialschwächung in X-Form in vier Lappen. Die Folie wird also vom Innendruck gesprengt, das Förderfluid 36 strömt unter seinem Druck durch den Blister, nimmt dabei das zweite Medium 37 mit, indem es sich mit diesem zur Bildung eines Festkörper-Aerosols vermischt. Durch einen in dem Adapter gebildeten Raum 44 strömt das Gemisch zur Austragöffnung 15 und wird an der gewünschten Stelle, an die der Benutzer es angesetzt hatte, beispielsweise in eine Nasenhöhle, appliziert.

Statt der gezielten Materialschwächung an bestimmten Stellen, kann diese auch in Form von Linien oder Punkten vorgesehen sein, die bereits der Folienhersteller vor der Blisterverarbeitung vornimmt. Auch sie bildet eine mechanische Vorschwächung, die eine Sollbruchstelle entstehen lässt. Dies trägt dazu bei, den Blister zwar "explodieren" zu lassen und damit eine schlagartige Aufwirbelung des Wirkstoffs in der zweiten Kammer zu bewirken, jedoch sollte schon wegen der Verschreckung des Benutzers ein Knall durch eine definierte Aufreißrichtung, d.h. eine stärkere Materialschwächung an einem Punkt und daran anschließende geringere "Weiterreiß"-Schwächung vermieden werden.

Es ist auch möglich, den Blister mit dem Adapter 14 baulich zu vereinigen, so dass diese Einheit für eine erneute Betätigung ausgetauscht wird. Damit wird sichergestellt, dass auch das ggf. mit Körperflüssigkeit in Berührung kommende Adapterteil mit ausgetauscht wird.

Es ist noch zu bemerken, dass vor dem Beginn der Betätigung das Betätigungselement einen Druckpunkt überwinden musste, der durch einen Steg 45 im Schlitz 32 gebildet wurde. Dieser Steg kann fest angeordnet sein, so dass die Rastnase 31 mit ihrer Schräge und der Aufweitung des Mantels, der gegebenenfalls durch Längsschlitze elastisch sein kann, den Druckpunkt erzeugt. Die Stege 45 können auch mit Sollbruchstellen versehene Abbrechstege sein, die einen genau vorgegebenen Widerstand erzeugen. Dadurch wird schon vor dem Beginn der Benutzung dem Benutzer ein gewisser Kraftaufwand abgefordert, der sicherstellt, dass die Betätigung nicht zu zaghaft und damit weniger wirksam erfolgt. Allerdings ist durch die Tatsache, dass die Öffnung des Überström-Verschlusselementes 41 (Blisterboden) erst nach Erzeugung des Drucks in der ersten Kammer erfolgt, von da an eine Automatik geschaffen, die für einen sicheren Austrag sorgt. Gegebenenfalls kann der Kolben auch noch nach dem Durchstechen des Blisterbodens weiter in Austragrichtung gedrückt werden und dabei ggf. auch die das Austragverschlusselement 22 bildende Folie anritzen, für den Fall, dass die Folie nicht allein vom Luftdruck aufgerissen wurde. Sie reißt dann aber schlagartig und großflächig auf, so dass ihre Öffnung und damit der Austrag des zweiten Mediums durch eine sehr große Öffnung erfolgt.

Durch die Betätigung der Pumpe wurde die Rückstellfeder 33 gespannt und führt das Betätigungselement 25 und damit den Pumpenkolben 27 wieder in seine Ausgangsstellung zurück, wodurch sich die Pumpkammer wieder mit Luft füllt. Durch Abnehmen des Adapters 14 und Auswechseln des Blisters 19 gegen einen unbenutzten wird der Spender wieder einsatzbereit. Im Falle, dass es sich um einen Einmal-Spender handelt, kann die Feder 33 entfallen.

Das Verhältnis des Pumpenhubvolumens zu einem Totraum im Abschnitt 18 einschließlich des Volumens der zweiten Kammer 19 bestimmt den Druck, der den vorbestimmten Berstdruck des Austragsverschlusselementes 22 überschreiten sollte.

Figur 4 zeigt einen Spender, dessen Basiskörper 12 mit Schultern 13 einen beispielsweise aus Glas bestehenden Zylinder 19a aufnimmt, der die zweite Kammer bildet und das zweite Medium 37 enthält. Der Zylinder 19 a ist beidseitig durch je einen Kolbenstopfen verschlossen, von denen der in Figur 4 untere das Überström-Verschlusselement 41 bildet und der obere das Austrag-Verschlusselement 22. Die Kolbenstopfen haben einen H-förmigen Längsschnitt mit einem dünneren mittleren Steg 46, der eine durchstechende Membran bilden kann, insbesondere beim Überström-Verschlusselement 41. Im übrigen laufen sie mit Dichtlippen auf der Zylinder-Innenfläche der zweiten Kammer 19a. Dieser hat im Bereich des Austrag-Verschlusselementes 22 einen Überströmkanal oder Bypass 48, der jedoch im dargestellten Ruhezustand zur zweiten Kammer hin durch das Austrag-Verschlusselement 22 verschlossen ist.

Der Basiskörper 12 führt ein hülsenförmiges Betätigungselement 25a, das in seinem Inneren die erste Kammer 16 bildet und das erste Medium 36 enthält. Die erste Kammer ist durch einen Kolben 49 begrenzt, in dessen Mitte mittels einer Buchse ein Stoßdorn 29 in Form einer Hohlnadel eingesetzt ist.

Der auch bei dieser Ausführungsform vorhandene Adapter 14 ist auf das obere Ende des Basisteils 12 aufgeschraubt und spannt einen oberen Flansch 50 der ersten Kammer 19 fest ein und drückt ihn in eine konische Aufnahme 51 im Basisteil.

Die Funktion ist ähnlich der, die unter 1 erläutert wurde:

Bei Druck auf die Betätigungsfläche 39 des Betätigungselements 25a wird dieses in das Basisteil 12 hineinverschoben. Der Stoßdorn 29 durchsticht den Steg 46 des Überströrn-Verschlusselementes 41 und öffnet dieses. Die untere Stirnfläche 52 der zweiten Kammer 19a trifft auf den Kolben 49 und drückt diesen nach unten, so dass das Förderfluid 36 durch die hohle Nadel 29 in die zweite Kammer 19a einströmt und sich mit dem zweiten Medium 37 mischt. Wenn der Druck in dieser Kammer so groß wird, dass er das Austrag-Verschlusselement 22 bildende obere Kolben weiter nach oben verschoben wird, wird dadurch der Austragverschluss geöffnet, und zwar durch den Bypass 48. Auch hier bewirkt der Innendruck in der zweiten Kammer 19a die Öffnung des Austragverschlusses. Die entstandene Mischung strömt dann in im wesentlichen linearer Durchströmung der zweiten Kammer 19a zur Austragöffnung 15. Bei dieser Ausführung ist die erste Kammer 16 zur Atmosphäre hin offen. Wenn das Förderfluid 36 Luft ist, ist dies kein Problem.

## Patentansprüche

1. Spender zum Austragen eines aus einem ersten und einem zweiten Medium (36, 37) gemischten Mediumsgemisch durch eine. Austrittsöffnung (15) des Spenders (11) mit einer das erste Medium (36) enthaltenen ersten Kammer (16) und einer das zweite Medium (37) enthaltenden zweiten Kammer (19, 19a) und mit einem Austrag-Verschlusselement (22) für die zweite Kammer (19, 19a) zwischen dieser und der Austrittsöffnung (15), wobei das Austrag-Verschlusselement (32) zu seiner Öffnung durch Mediendruck in der zweiten Kammer (19, 19a) ausgebildet ist, und wobei der Medienverbindungspfad zwischen erster und zweiter Kammer (16; 19, 19a) von einem Überström-Verschlusselement (41) verschlossen ist, **dadurch gekennzeichnet, dass** das Überström-Verschlusselement (41) mittels eines Stoßdorns (29) durchstechbar ist, der zusammen mit einem Betätigungselement (25) für eine Druckerzeugung in der ersten Kammer (16) betätigbar ist.

2. Spender nach Anspruch 1, **dadurch gekennzeichnet, dass** die zweite Kammer (19, 19a) ein Blister mit einem Formkörper (20) und einer diesen verschließenden Folie ist.

3. Spender nach Anspruch 1, **dadurch gekennzeichnet, dass** das Austrag-Verschlusselement einen axial verschiebbar angeordneten Verschlusskörper aufweist, das mittels eines den Verschlusskörper umgehenden Überströmkanals (48) zu öffnen ist.

4. Spender nach einem der Ansprüche 1 bis 3, **dadurch gekennzeichnet, dass** die zweite Kammer (19a) als im wesentlichen zylindrischer Körper ausgebildet ist, der beiderseits mit einem stopfenartigen Verschlusskörper verschlossen ist, wobei der eine Verschlusskörper das Austrag-Verschlusselement (22) bildet und der andere Verschlusskörper das Überström-Verschlußelement (41), das mittels des ggf. hohlen Stoßdorns (29) durchstechbar ist.

5. Spender nach einem der Ansprüche 1 bis 4, **dadurch gekennzeichnet, dass** die zweite Kammer (19) austauschbar im Spender (11) aufgenommen ist.

6. Spender nach einem der vorhergehenden Ansprüche, **dadurch gekennzeichnet, dass** das Austrag-Verschlusselement (22) eine Materialschwächung (42), insbesondere durch eine Sollbruchstelle, eine verringerte Materialstärke oder eine Laserperforierung in einer Wandung der zweiten Kammer (19) enthält

7. Spender nach einem der vorhergehenden. Ansprüche, **dadurch gekennzeichnet, dass** die Materialschwächung durch Anstechen der Wandung mittels des Stoßdorns (29) erzeugt wird, der auch das Oberström-Verschlusselement (41) öffnet.

8. Spender nach einem der vorhergehenden Ansprüche, **dadurch gekennzeichnet, dass** das Austrag-Verschlusselement (22) durch Verschieben eines Verschlusskörpers geöffnet wird.

## Claims

1. Dispenser for discharging a medium mixture mixed from a first and a second medium (36, 37) through a discharge opening (15) of dispenser (11) with a first chamber (16) containing the first medium (36) and a second chamber (19, 19a) containing the second medium (37) and with a discharge closure element (22) for the second chamber (19, 19a) between the latter and the discharge opening (15), the discharge closure element (32) being constructed for the opening thereof by medium pressure in the second chamber (19, 19a), and the media connecting path between the first and second chambers (16, 19, 19a) being closed by an overflow closure element (41), **characterized in that** the overflow closure element (41) is perforatable by means of an impact spike (29) actuatable together with an actuator (25) for producing pressure in the first chamber (16).

2. Dispenser according to claim 1, **characterized in that** the second chamber (19, 19a) is a blister with a moulding (20) and a foil sealing the same.

3. Dispenser according to claim 1, **characterized in that** the discharge closure element has an axially displaceably arranged closure body, which can be opened by an overflow channel (48) bypassing the closure body.

4. Dispenser according to one of the claims 1 to 3, **characterized in that** the second chamber (19a) is constructed as a substantially cylindrical body bilaterally closed by a plug-like closure body, one closure body forming the discharge closure element (22) and the other closure body the overflow closure element (41) which is perforatable by means of an optionally hollow impact spike (29).

5. Dispenser according to one of the claims 1 to 4, **characterized in that** the second chamber (19) is replaceably received in the dispenser (11).

6. Dispenser according to any one of the preceding claims, **characterized in that** the discharge closure element (22) is formed by a material weakening (42), particularly by a predetermined breaking point, a reduced material thickness or a laser perforation in a wall of the second chamber (19).

7. Dispenser according to any one of the preceding claims, **characterized in that** the material weakening is produced by piercing the wall by means of an impact spike (29), which preferably also opens the overflow closure element (41).

8. Dispenser according to any one of the preceding claims, **characterized in that** the discharge closure element (22) is opened by displacement of a closure member.

## Revendications

1. Distributeur pour décharger un mélange de substances, composé d'une première et d'une deuxième substance (36, 37), à travers une ouverture de sortie (15) du distributeur (11), avec une première chambre (16) contenant la première substance (36) et une deuxième chambre (19, 19a) contenant la deuxième substance (37), et avec un élément de fermeture de décharge (22) pour la deuxième chambre (19, 19a) entre celle-ci et l'ouverture de décharge (15), sachant que l'élément de fermeture de décharge (32) est réalisé de manière à pouvoir être ouvert par la pression de substance dans la deuxième chambre (19, 19a), et sachant que la voie de raccordement de substances entre la première et la deuxième chambre (16 ; 19, 19a) est fermée par un élément de fermeture de débordement (41), **caractérisé en ce que** l'élément de fermeture de débordement (41) peut être percé au moyen d'un poinçon (29) qui peut être actionné conjointement avec l'élément d'actionnement (25) pour générer une pression dans la première chambre (16).

2. Distributeur d'après la revendication 1, **caractérisé en ce que** la deuxième chambre (19, 19a) est un blister avec un corps moulé (20) et une pellicule qui referme celui-ci.

3. Distributeur d'après la revendication 1, **caractérisé en ce que** l'élément de fermeture de décharge présente un corps de fermeture disposé de manière axialement déplaçable, qui peut être ouvert au moyen d'une conduite de trop-plein (48) contournant le corps de fermeture.

4. Distributeur d'après une des revendications de 1 à 3, **caractérisé en ce que** la deuxième chambre (19a) est réalisée en tant que corps essentiellement cylindrique, qui est fermé des deux côtés par un corps de fermeture genre bouchon, sachant que l'un des corps de fermeture constitue l'élément de fermeture de décharge (22) et l'autre corps de fermeture l'élément de fermeture de débordement (41), qui peut être percé au moyen du poinçon (29) éventuellement creux.

5. Distributeur d'après une des revendications de 1 à 4, **caractérisé en ce que** la deuxième chambre (19) est logée dans le distributeur (11) de manière remplaçable.

6. Distributeur d'après une des revendications précédentes, **caractérisé en ce que** l'élément de fermeture de décharge (22) comprend un affaiblissement du matériau (42), notamment par un point de rupture intentionnellement prévue, une réduction d'épaisseur du matériau ou bien une perforation par laser dans une paroi de la deuxième chambre (19).

7. Distributeur d'après une des revendications précédentes, **caractérisé en ce que** l'affaiblissement du matériau (42) est obtenu en piquant la paroi au moyen du poinçon (29), qui ouvre aussi l'élément de fermeture de débordement (41).

8. Distributeur d'après une des revendications précédentes, **caractérisé en ce que** l'élément de fermeture de décharge (22) est ouvert par le déplacement d'un corps de fermeture.
